# EUROPÄISCHE PATENTSCHRIFT

(11) **EP 1 512 430 B1**
(45) Veröffentlichungstag und Bekanntmachung des Hinweises auf die Patenterteilung: **13.02.2008**
(21) Anmeldenummer: 04090229.8
(22) Anmeldetag: 10.06.2004
(51) Int. Cl.: A61N 1/365, A61B 5/11

(54) **Vorrichtung zur Behandlung von Schlaf-Apnoe**
Device for sleep-apnea treatment
Dispositif de traitement des apnées du sommeil

(30) Priorität: 02.09.2003 US 499631 P; 02.10.2003 DE 10347294
(43) Veröffentlichungstag der Anmeldung: 09.03.2005
(73) Patentinhaber: Biotronik GmbH & Co. KG, 12359 Berlin (DE)
(72) Erfinder: Hsu, William, West Linn, OR 97068 (US); Johnson, Mark, Aurora, OR 97002 (US); Czygan, Gerald, 91054 Buckenhof (DE)
(74) Vertreter: Lindner-Vogt, Karin L.

(56) Entgegenhaltungen:
- EP-A- 0 515 319
- US-A- 4 846 195
- US-A- 4 869 251
- US-A- 4 926 863
- US-A- 5 562 711
- US-A- 6 083 248
- US-A1- 2002 193 939
- US-A1- 2003 130 589
- US-A1- 2003 153 953
- US-B1- 6 574 507

## Beschreibung

Die Erfindung betrifft ein medizinisches Gerät zur Implantation in einen Körper, mit einer Stimulationseinheit, welche ausgebildet ist, in Abhängigkeit von einem Apnoe-Therapiesignal einen elektrischen Stimulationsimpuls zu erzeugen, einer Schlaf-Detektoreinheit mit mindestens einem Signaleingang, welche ausgebildet ist, in Abhängigkeit von mindestens einem Eingangssignal einen Schlafzustand des Körpers zu erkennen und ein Schlaf-Signal zu erzeugen, welches einem Schlaf-Erkennungsergebnis entspricht. Das medizinische Gerät zur Implantation in einen Körper, im Folgenden auch implantierbares medizinisches Gerät genannt, weist auch eine Apnoe-Detektoreinheit auf, welche ausgebildet ist, in Abhängigkeit von mindestens einem von dem Körper verursachten Körpersignal eine Schlaf-Apnoe zu erkennen und ein Apnoe-Signal zu erzeugen, welches einem Apnoe-Erkennungsergebnis entspricht. Das implantierbare medizinische Gerät weist auch eine Therapieeinheit auf, welche mit der Schlaf-Detektoreinheit und mit der Apnoe-Detektoreinheit mindestens mittelbar verbunden und ausgebildet ist, in Abhängigkeit von dem Apnoe-Signal und dem Schlaf-Signal mindestens ein Apnoe-Therapiesignal zu erzeugen, welches Therapieinformation wahlweise zur Prävention von Schlaf-Apnoe, zur Behandlung von Schlaf-Apnoe oder beidem repräsentiert und dieses an die Stimulationseinheit zu senden.

Ein solches System hat einen besonderen Vorteil für Patienten, bei denen bereits eine Herzschrittmacher-Implantation angezeigt ist. Ein solches System kann auch vorteilhaft sein für Patienten, welche unter Schlaf-Apnoe leiden, ohne dass andere Indikationen für eine Implantation vorliegen. Ein signifikanter Bevölkerungsanteil ist von Atmungsstörungen betroffen. Eine solche Atmungsstörung ist beispielsweise Schlaf-Apnoe, ein vorübergehendes Aussetzen der Atmung. Die häufigste Erscheinung ist die obstruktive Schlaf-Apnoe, von welcher näherungsweise 6% der männlichen Bevölkerung in einem Alter über 40 Jahren betroffen sind. Bei der obstruktiven Schlaf-Apnoe kollabieren die oberen Luftwege und verschließen, wodurch das Passieren von Atemluft verhindert wird. Dies kann wiederholt im Schlaf auftreten.

Eine andere Erscheinungsform der Schlaf-Apnoe ist das zentrale Schlaf-Apnoe-Syndrom. Bei diesem Syndrom bleiben die Atemwege offen, aber die zentrale Kontrolle der Atemmuskulatur ist nachteilig beeinflusst. Während diese Form der Apnoe bei etwa 10 bis 20% von allen an Schlaf-Apnoe Leidenden beobachtet wird, hat sie ein großes Übergewicht bei Patienten mit Herzinsuffizienz. Solche Patienten haben auch die Cheyne-Stokes-Atmung, welche ein periodisches Abnehmen und Zunehmen der Atemamplitude ist, auch als Atemtiefe bezeichnet. Der Patient hat in diesem Fall Perioden geringerer Atemtiefe, das bedeutet, zentrale Apnoe, und Perioden gesteigerter Atemtiefe, auch als Hyperventilation bezeichnet. Von diesen Atemfehlfunktionen sind die Herzrate, die Hämodynamik und der Blutdruck beeinflusst. Beispielsweise können die Apnea-Perioden eine Zunahme der sympathischen Aktivität anregen, welche das Herz negativ beeinträchtigen kann. Das Zusammenwirken von Schlaf-Apnoe mit Herzinsuffizienz reduziert wesentlich die Lebensqualität und die Leistungsfähigkeit des Patienten. Daher ist es wesentlich, dass in solchen Fällen die Schlaf-Apnoe überwacht, identifiziert und behandelt wird.

Im Allgemeinen erfordern Atemfehlfunktionen, wie beispielsweise die Schlaf-Apnoe, eine kontinuierliche Überwachung eines Patienten und wenn mögliche eine kontinuierliche Behandlung, eher als nur während eines Aufenthaltes in einer medizinischen Einrichtung. Um diese Fehlfunktion zu überwachen und zu behandeln, muss der Patient während des Schlafes zu Hause überwacht und behandelt werden. Geräte aus dem Stand der Technik schließen die Verwendung von extern angebrachten Atemsensoren und Atemmasken ein. Bei diesen Geräten wird die Schlaf-Apnoe durch künstliche Atemgeräte behandelt, welche die Atmung die kontrollieren und eine Einatmung und Ausatmung erzwingen. Tatsächlich bedeuten diese Geräte eine signifikante Einschränkung der Lebensqualität. Da diese Therapie von der Kooperation des Patienten abhängt, könnte die aufdringliche Natur dieses Gerätes seine laufende Verwendung verhindern.

In dem Artikel "Benefit of atrial pacing in sleep apnea syndrome" von Garrigue, et al., erschienen im Magazin New England Journal of Medicine (vol. 346, Nr. 6, pp. 404 - 412, 7. Februar 2002) ist eine Studie beschrieben, welche auf eine Patientengruppe angewendet wurde, welche bereits implantierte Herzschrittmacher zur Behandlung einer Sinusbradykardie mit Hilfe atrialer Übererregung besaßen. Basierend auf einigen Berichten dieser Patienten, dass sie nach der Implantation weniger Atemschwierigkeiten hatten, wurden 15 Patienten zur Untersuchung in einer Folge von Nächten ausgewählt. Während des Tests wurde der implantierte Schrittmacher programmiert, das Herz entweder gar nicht oder durch kontinuierliche Impulse zu erregen. Während der Erregungsphase wurde eine atriale Übererregung von etwa 15 Schlägen pro Minute über der durchschnittlichen Herzrate bei Nacht des Patienten eingestellt. Bei 13 von 15 Patienten sank der beobachtete Apnoe-Hypopnea-Index um mehr als 50% während der Nächte mit kontinuierlicher Zwei-Kammer-Erregung durch den Herzschrittmacher. Der Apnea-Hypopnea-Index ist eine Messung der Frequenz der gestockten oder verlangsamten Atemfrequenz bei Nacht. Der Grund für die beobachtete Verbesserung ist nicht zitiert, aber durch die Schrittmacherapparatur konnte sowohl die obstruktive als auch die zentrale Apnoe verbessert werden.

Ende des Jahres 2003 hat das St. Jude Medical Center eine neue Studie zur Evaluierung einer Schrittmachertherapie für Schlaf-Apnoe veröffentlicht. Wie bei der Garrigue-Studie, war hier der Plan den Einfluss einer erhöhten Schrittmacherrate während einer Ruhe-Phase zu bewerten, aber die Studie ist offensichtlich auf Herzschrittmacher-Patienten mit diagnostizierter Schlaf-Apnoe beschränkt. Offensichtlich will die St. Jude-Studie einen geheimen Algorithmus zum Herunterladen in den Herzschrittmacher eines Patienten verwenden.

Aus US 2003/0153953 ist ein implantierbarer Herzstimulator bekannt, welcher in der Lage ist, eine Schlaf-Apnoe zu detektieren und daraufhin die Grundstimulationsrate anzuheben.

Aus US 4,846,195 ist ein Herzschrittmacher bekannt, der einen Positions- und Bewegungssensor zur Anpassung der Stimulationsrate an den physiologischen Bedarf des Patienten aufweist.

In der Schrittmacher-Therapie ist die Verwendung des Herzschlagvolumens als Eingabeparameter zur Justierung oder Anpassung der Schrittmacher-Rate bekannt. Ein Atemvolumen-Wert wird aus der Frequenz und der relativen Amplitude eines Atem-Signals berechnet, welches aus einer Messung einer intrathorakalen Impedanz bestimmt werden kann. Offensichtlich verwendet der St. Jude-Algorithmus eine Tageszeit-Uhr, um die Schrittmacher-Therapie ein- und auszuschalten. Selbstverständlich kann ein Algorithmus, basierend auf dem Auslesen einer Tageszeit-Uhr Probleme bereiten, wenn der Patient beim Reisen Zeitzonen passiert, in Folge dessen Zeitwechsel eintreten (wie auch solche Zeitwechsel zur Gewinnung von Tageslichtzeit) und wenn der Patient Schlafrhythmusstörungen hat.

Es ist daher die Aufgabe der vorliegenden Erfindung, ein Gerät zur Behandlung von Schlaf-Apnoe mittels einer übersteuerten Erregung des Vorhofes eines Herzens mittels eines implantierten Herzschrittmachers anzugeben.

Diese Aufgabe wird durch die in Anspruch 1 angegebene Merkmale gelöst.

In einer bevorzugten Ausführungsform enthält die Apnoe-Detektoreinheit einen Apnoe-Gewichtungs-Diskriminator mit mindestens einem Eingang für ein Körpersignal enthält. Der Apnoe-Gewichtungs-Diskriminator ist ausgebildet, gemäß einer vorbestimmten Apnoe-Gewichtungsfunktion das Körpersignal zu bewerten und ein eine Schlaf-Apnoe repräsentierendes Apnoe-Signal zu erzeugen.

Die Schlaf-Detektoreinheit enthält bevorzugt einen Schlaf-Gewichtungs-Diskriminator mit mindestens einem Eingang für ein Eingangssignal, wobei der Schlaf-Gewichtungs-Diskriminator ausgebildet ist, gemäß einer vorbestimmten Schlaf-Gewichtungsfunktion das Eingangssignal zu bewerten und ein einen Schlafzustand repräsentierendes Schlaf-Signal zu erzeugen.

Weiter bevorzugt enthält der Schlaf-Gewichtungs-Diskriminator und/oder der Apnoe-Gewichtungs-Diskriminator eine Fuzzy-Logik.

Das medizinische Gerät enthält bevorzugt eine Tageszeit-Uhr, welche mit der Schlaf-Detektoreinheit verbunden ist und somit als ein Eingangs-Parameter zur Entscheidung, ob ein Schlaf-Status besteht oder nicht, zur Verfügung steht.

In einer besonderen Ausführungsform weist das medizinische Gerät einen Welt-Zeitzonen-Detektor mit einem Satellitenempfänger auf, wobei der Welt-Zeitzonen-Detektor mit der Tageszeit-Uhr wirkverbunden und ausgebildet ist, Satellitensignale zu empfangen, durch Auswertung der Satellitensignale eine Position auf der Erde zu ermitteln und anhand der Position auf der Erde ein Welt-Zeitzonensignal zu erzeugen und dieses an die Tageszeit-Uhr zu senden, welche ausgebildet ist, die Tageszeit entsprechend zu korrigieren.

Beispielsweise kann das Satellitenempfangssystem die Position in Form von Koordinaten bestimmen. Anhand der Koordinaten kann das Satellitenempfangssystem eine Zuordnung in eine Welt-Zeitzone treffen, in welcher sich der Träger des medizinischen Gerätes derzeit befindet. Das Satellitenempfangssystem ist bevorzugt mit der Tageszeit-Uhr verbunden und kann somit eine Korrektur der Tageszeit bewirken, wenn der Träger des medizinischen Gerätes sich in einer anderen Zeitzone befindet. Dadurch kann vorteilhaft auf einfache Weise sichergestellt werden, dass der Träger des medizinischen Geräts bei Reisen durch verschiedene Zeitzonen der Welt nicht zur falschen Zeit therapiert wird, sofern eine Therapie in Abhängigkeit von einer Tageszeit erfolgen soll. Bevorzugt enthält das Satellitenempfangssystem das aus dem Stand der Technik bekannte Global-Positioning-System (GPS).

Die Stimulationseinheit ist ausgebildet, einen elektrischen Stimulationsimpuls zu erzeugen, welcher geeignet ist, um ein Gewebe derart zu stimulieren, dass eine Depolarisationsschwelle für eine Muskelkontraktion überschritten wird.

In einer Ausführungsform weist die Stimulationseinheit Atemmuskulatur-Stimulationseinheit auf, welche ausgebildet ist, einen elektrischen Stimulationsimpuls zur Stimulation des Zwerchfells oder der Thoraxmuskulatur zu erzeugen. In dieser Ausführungsform weist das implantierbare medizinische Gerät einen Ausgang zum Anschluss von Atemmuskulatur-Stimulationselektroden auf.

In einer bevorzugten Ausführungsform weist das implantierbare medizinische Gerät einen Herzschrittmacher oder Defibrillator auf. In dieser Ausführungsform kann die Therapieeinheit mit dem Herzschrittmacher oder Defibrillator verbunden sein und an diesen Stimulationsinformation enthaltende Therapiesignale senden. Der Herzschrittmacher ist somit ausführendes Mittel zur Therapierung von Schlaf-Apnoe.

Ein Ausführungsbeispiel für einen Aktivitätssensor ist ein Beschleunigungsaufnehmer oder eine Closed-Loop-Stimulation (CLS).

Ein Ruhe- oder Lastzustand des Körpers wird bei einer Closed-Loop-Stimulation aus einem intrakardialen Impedanzsignal abgeleitet. Diese Technik ist in dem Artikel "Closed Loop stimulation - Ein neues Herzschrittmacher - Konzept zur Frequenzadaption mittels eines Kontraktilitätssensors" im Journal Kardiol, 1999, Vol. 6, Iss.1, S. pp. 21-25 erläutert.

Das implantierbare medizinische Gerät weist in einer bevorzugten Ausführungsform einen Sensor zur Erfassung der intrakardialen Impedanz auf, welcher mit dem Schlafdetektor verbunden und ausgebildet ist, anhand der intrakardialen Impedanz ein Ruhen des Körpers zu erkennen und ein den Ruhezustand des Körpers repräsentierendes Ruhezustands-Signal zu erzeugen. Der Sensor zur Erfassung der intrakardialen Impedanz kann auch als Kontraktilitätssensor ausgebildet sein.

Das medizinische Gerät kann bei Geräten mit CLS den intrakardialen Impedanzsensor der CLS vorteilhaft mitnutzen, um ein intrakardiales Impedanzsignal aus der CLS abzuzweigen.

Ein Beschleunigungsaufnehmer kann vorteilhaft als triaxialer Beschleunigungsaufnehmer ausgebildet sein, bei welchem die Richtungsachsen der erfassbaren Beschleunigungen ein Orthogonalsystem bilden und welcher mit dem Schlaf-Detektor wirkverbunden und ausgebildet ist, ein eine Beschleunigung repräsentierendes Beschleunigungs-Zeitsignal zu erzeugen.

Dadurch kann eine Körperbewegung derart ausgewertet werden, dass das implantierbare medizinische Gerät bei einer Auswertung der Zeitsignale des Beschleunigungsaufnehmers anhand bevorzugt auftretender Beschleunigungsrichtungen zwischen normalen Tagesbewegungsabläufen und beispielsweise Rollbewegungen während eines Schlafes unterscheiden kann.

Das implantierbare medizinische Gerät weist in dieser Ausführung bevorzugt einen Beschleunigungsmuster-Klassifizierer auf, welcher ausgebildet ist, anhand vorbestimmter Beschleunigungsmuster, welche jeweils für alle drei Bewegungsachsen abgespeichert sein können, Körperbewegungen während eines Schlafes von denen während eines Tagesablaufes zu unterscheiden.

In dieser Ausführungsform ist der Beschleunigungsmuster-Klassifizierer ausgangsseitig mit der Schlaf-Detektoreinheit und eingangsseitig mit dem Beschleunigungsaufnehmer verbunden und ausgebildet, ein Beschleunigungs-Zeitsignal auszuwerten und vorbestimmte Beschleunigungsmuster in dem Beschleunigungs-Zeitsignal zu erkennen, diese zu klassifizieren und ein ein Beschleunigungsmuster repräsentierendes Beschleunigungsmuster-Signal zu erzeugen und dieses ausgangsseitig auszugeben.

Alternativ zu einem Beschleunigungsaufnehmer kann das Medizinische Gerät auch einen Geschwindigkeitsaufnehmer aufweisen.

Die zur Klassifizierung vorgesehenen Beschleunigungsmuster können Zeitsignale oder Leistungsspektren sein, im Falle der Leistungsspektren weist der Beschleunigungsmuster-Klassifizierer eingangsseitig einen FFT-Analysator (FFT: Fast-Fourier-Transformation) auf, welcher ausgebildet ist, aus einem Beschleunigungs-Zeitsignal kontinuierlich Beschleunigungsmuster-Leistungsspektren zu erzeugen und diese an den Beschleunigungsmuster-Klassifizierer zu senden. Die Abspeicherung von Leistungsspektren zum Klassifizierungsvergleich ist im Vergleich zur Abspeicherung von Zeitsignalen vorteilhaft besonders sparsam im Speicherplatzverbrauch.

In einer bevorzugten Ausführungsform weist das implantierbare medizinische Gerät eine Tageszeit-Uhr auf, welche mit der Schlaf-Detektoreinheit verbunden ist.

Weiter bevorzugt weist das implantierbare medizinische Gerät zusätzlich zur Tageszeit-Uhr mindestens einem Aktivitäts-Sensor auf, um die Genauigkeit der Erkennung zu steigern, ob ein Schlaf begonnen hat oder endet und ob eine Stimulationstherapie gestartet oder beendet werden sollte.

Um den Stimulationsalgorithmus der vorliegenden Erfindung zu aktivieren, das bedeutet, das Stimulationsprotokoll auf "An" zu schalten, erfordert der Algorithmus, dass die Tageszeit-Uhr anzeigen muss, dass die Zeit in einem vorbestimmten Zeitintervall zum Schlafen liegt und dass mindestens ein Aktivitätssensor das Fehlen einer Bewegung des Patienten anzeigt.

Ein ersichtliches Anliegen ist die Fähigkeit des Patienten, beim Einschalten des Stimulationsprotokolls einzuschlafen, welches auftreten kann, wenn das Protokoll durch die oben genannten Bedingungen aktiviert ist. Das Stimulationsprotokoll bedeutet in diesem Fall ein Ansteigen der Herzrate. Hierzu ist erfindungsgemäß eine Zunahme der Stimulationsrate mit einer ansteigenden Flanke über eine Periode von 10 Minuten vorgesehen. Die Gesamtzunahme der Stimulationsrate beträgt 10 Schläge pro Minute und wird durch einen Anstieg der Stimulationsrate von einem Schlag pro Minute über 10 Minuten erreicht.

Der Schlafalgorithmus der vorliegenden Erfindung kann das Stimulationsprotokoll zu Aus" setzen, wenn das vorbestimmte Zeitintervall erreicht ist und eine Bewegung von mindestens einem Aktivitätssensor erkannt ist. Ebenso wie ein bevorzugter Schlafalgorithmus die Stimulationsrate gemäß eine Rampenfunktion beim Anschalten des Stimulationsprotokolls steigern kann, kann der bevorzugte Schlafalgorithmus die Stimulationsrate auch auf eine ähnliche absolute Rate absenken. Wenn zum Beispiel die Stimulationsrate beim Einschalten um einen Schlag pro Minute zunimmt, sollte die Abnahme auch einen Schlag pro Minute beim Beenden betragen.

Wenn mehr als nur ein Aktivitätssensor vorhanden ist, sind eine Vielzahl von Algorithmen zur Erkennung vorgesehen, ob ein "KEINE-BEWEGUNG-Status" erreicht ist. Andernfalls ist eine positive Erkennung des "KEINE-BEWEGUNG-Status" einer der vorgenannten Abfühlmittel ausreichend. In einem weiter bevorzugten dritten Algorithmus kann den verschiedenen Abfühlmitteln jeweils ein Gewichtungsfaktor und das Vorherrschen von "KEINE-BEWEGUNG" gewichteten Signalen zugewiesen werden, was ausreichend sein kann.

Bei jedem der Schlafalgorithmen der vorliegenden Erfindung ist die Möglichkeit, das Ansteigen/Abfallen der Stimulationsrate und die vorbestimmte Schlafzeitperiode extrakorporal zu programmieren, als wesentlich erachtet.

In einigen Ausführungsformen der vorliegenden Erfindung liest der Algorithmus auch das Atemmuster des Patienten über die Signalform der Minutenventilation oder die Closed-Loop-Stimulation (CLS) Signalform, um eine Schlaf-Apnoe zu erkennen. In dieser Ausführungsform kann eine Stimulation nur über die Erkennung einer Schlaf-Apnoe auf "An" geschaltet werden.

In einigen weiteren Ausführungsformen ist ein einfacher Positionsdetektor ausgebildet, wobei in diesem Fall der Status des Positionsdetektors in einem Teil des Schlafalgorithmus verwendet werden kann, um zu erkennen, ob ein Schlaf begonnen hat.

Ein implantierbares Monitoring-System der vorliegenden Erfindung überwältigt alle Grenzen bisher bekannter Systeme. Das System kann die Atmung überwachen und Atmungsinformationen mit anderen diagnostischen Daten zu einem Remote-Monitoring-Center senden. In dieser Weise ist ein Arzt in der Lage, den Patienten zu überwachen, ohne den Patienten kontinuierlich zu beobachten. Der Atmungsmonitor kann in ein therapeutisches Gerät integriert sein, welches ein implantierbarer Schrittmacher oder ein Defibrillator (ICD, ICD: Implantable Cardioverter/Defibrillator) ist, oder es kann als Stand-Alone diagnostisches Gerät arbeiten.

Die Atmung wird über die intrathorakale Impedanz gemessen.

Eine Überwachung kann mit verschiedenen therapeutischen Mitteln kombiniert sein. Beispielsweise kann eine Schlaf-Apnoe-Therapie in eine Resynchronisations-Therapie unter Verwendung eines Herzschrittmachers oder Defibrillators (ICD) für Patienten mit Herzinsuffizienz eingebunden sein.

Der implantierbare Atmungsmonitor umfasst Mittel zur Messung der Impedanz, ein Mittel für eine Long-Distance-Telemetrie (LDT), Mittel zum Speichern von Daten, Mittel zum Messen eines Herzaktionssignales und eine zentrale Steuereinheit. Viele der Monitore können auch ein Elektrotherapeutisches Modul umfassen, wie einen Herzschrittmacher, einen Defibrillator (ICD) oder beides.

In einer Ausführungsform können die Mittel zur Messung der Impedanz einen unter-reizschwelligen elektrischen Strom zwischen zwei Elektroden, welche von den zur Verfügung stehenden Leitungen ausgewählt sind, injizieren. Der Strom kann aus biphasigen Pulsen mit konstanter Amplitude bestehen. Zur Messung einer Spannung kann ein Elektrodenpaar verwendet werden.

Die gemessene Spannung ist in diesem Fall proportional zur Impedanz des Gewebes in der Messregion. In einigen Ausführungsformen sind die Strom- und Spannungselektroden dieselben Elektroden. Vor einer Analog-DigitalWandlung wird die Spannung verstärkt und gefiltert. Eine Bandpassfilter ist hierfür bevorzugt. Der Filter kann so ausgewählt sein, dass das Atmungssignal passiert, aber höhere und niedrigere Frequenzkomponenten abgeschwächt sind.

Das Medizinische Gerät weist bevorzugt einen Sauerstoffsensor auf, welcher mit der Apnoe-Detektoreinheit verbunden und ausgebildet ist, eine Sauerstoffkonzentration in Blut zu bestimmen und ein die Blut-Sauerstoffkonzentration repräsentierendes Blut-Sauerstoffsignal zu erzeugen. Die Sauerstoffkonzentration in Blut dient somit als Eingangsgröße für die Apnoe-Detektoreinheit.

In einer Ausführungsform weist das implantierbare medizinisches Gerät eine Atemminutenvolumen-Erfassungseinheit auf, welche mit der Apnoe-Detektoreinheit verbunden ist. Bevorzugt enthält die Atemminutenvolumen-Erfassungseinheit Mittel zur Bestimmung der thorakalen Impedanz.

Um eine Atmung zu erkennen, muss die thorakale Impedanz ermittelt werden. Die Impedanz des Thorax verändert sich während der Atmungszyklen, weil die elektrische Leitfähigkeit der Lungen sich entsprechend einem unterschiedlichen Luftgehalt verändert. Die thorakale Impedanz ändert sich auch entsprechend geometrischen Unterschieden.

Eine bevorzugte Ausführung für einen Detektor der thorakalen Impedanz ist eine Tripolare-Messkonfiguration nutzt das Gehäuse oder die Dose des Implantats als gemeinsame Elektrode für Strom und Spannung. Zwischen der Dose und einer Ringelektrode der rechten ventrikulären Leitung oder der linken ventrikulären Leitung wird ein Strom injiziert. Die resultierende Spannung wird zwischen der Elektrodenspitze und der Dose gemessen, so dass gewissermaßen die Impedanz des thorakalen Gewebes gemessen wird. Auch eine Leitung zur Stimulation des linken Ventrikels, entweder eine Koronar-Sinus-Leitung oder Epikard-Leitungen können mit der entsprechenden tripolaren Konfiguration verwendet werden. In diesem Fall ist der linke Teil der Lunge in der Messregion enthalten. In einigen Ausführungsformen kann auch eine Defibrillatorleitung (ICD-Leitung), eingeschlossen der Erregungsspule, für Impedanzmessungen zum Einsatz kommen.

In einer Ausführungsform weist das Medizinische Gerät eine Herzschlagvolumen-Erfassungseinheit auf, welche mit der Schlaf-Detektoreinheit und/oder der Therapieeinheit verbunden und ausgebildet ist, ein Herzschlagvolumen zu bestimmen und ein das Herzschlagvolumen repräsentierendes Herzschlagvolumensignal zu erzeugen. Das Herzschlagvolumen kann somit als Eingangsgröße zur Schlaferkennung oder als Ist-Zustandsgröße für die Therapieeinheit zur Regelung des Herzschlagvolumens dienen.

In einer bevorzugten Ausführungsform umfasst der Positionssensor wenigstens zwei Schaltkontakte und mindestens eine elektrisch leitfähige Kugel welche derart angeordnet ist, in Abhängigkeit von der Neigung des Positionssensors die Schaltkontakte elektrisch leitend zu verbinden oder zu trennen. Die elektrisch leitfähigen Kugeln sind bevorzugt Metallkugeln.

Alternativ dazu kann ein Positionssensor anstatt der Metallkugeln auch Kohlekugeln enthalten. Bevorzugt enthalten die Kohlekugeln komprimierten Aktivkohlestaub, weiter bevorzugt zusätzlich ein Bindemittel. Der Vorteil des Einsatzes der Aktivkohlekugeln besteht darin, dass die Zahl der am Kurzschluss beteiligten Kohlekugeln vom Positionswinkel des Positionssensors gegenüber der horizontalen abhängt. Dadurch ergibt sich in Abhängigkeit von der Winkellage ein unterschiedlicher elektrischer Widerstand zwischen den Kontakten des Positionssensors. Alternativ kann zur Erzielung der gleichen Wirkung wie die der Kohlekugeln auch ein Kugelmaterial mit einem vorbestimmten ohmschen Widerstand zum Einsatz kommen. Bevorzugt sind solche Kugeln Kunststoffkugeln oder Glaskugeln, welche mit einer dünnen Metallschicht bedampft sind. Die Leitfähigkeit der Metallschicht ist über die Schichtdicke und die texturellen Eigenschaften einstellbar.

Der Positionssensor kann auch eine Hallsonde sein, welche ausgebildet ist, in Abhängigkeit von ihrer Ausrichtung im Erdmagnetfeld eine Hallspannung zu erzeugen.

Alternativ zu dieser Ausführungsform des Positionssensors kann ein Positionssensor im Sinne der Erfindung auch ein aus dem Stand der Technik bekannter Quecksilberschalter sein.

Das Fernbeobachtungssystem, welches mit der vorliegenden Erfindung zum Einsatz kommen kann, ist aus dem Stand der Technik bekannt. Das Implantat kann diagnostische Daten über die Long-Distance-Telemetrie-Mittel (LDTM) zu einem Gerät außerhalb des Patienten senden, typischerweise ein Gerät, welches seitlich eines Patientenbettes positioniert ist. Von dort werden die Daten zu einem zentralen Service-Center gesendet, wo ein Arzt auf die Daten zugreifen kann.

Das Atmungssignal eines Patienten stellt viele Parameter bereit, welche für diagnostische Zwecke extrahiert werden können. Diese Daten sind in dem Implantat gespeichert und können in einer komprimierten Form gesendet werden. Die gespeicherten Werte können auch mit einem externen Gerät vom Arzt abgefragt werden. Das externe Gerät kann die empfangenen Daten auf einem Display in Form von Zahlen, Trends, Histogrammen oder vergleichbaren Anzeigen. Für ein Fernüberwachung werden die Daten in Langzeit-Mittelwerte, Zahlenwerte usw. komprimiert, welche beispielsweise alle 24h zu einem Service-Center oder einer Normal-Basis gesendet werden. Zusätzlich kann das Gerät derart ausgestattet sein, dass es dem Patienten erlaubt ist, eine Sendung zu veranlassen. Das beabsichtige erfindungsgemäße Gerät kann auch mit Schwellwertschaltern ausgestattet sein, basierend auf verschiedenen möglichen Alarmkriterien.

Verschiedene diagnostische Parameter können aus dem Atmungssignal extrahiert und als Apnoe-Statistikinformation abgespeichert werden. Das können die folgenden Parameter sein, ohne auf diese beschränkt zu sein:

Atmungsfrequenz; Atemminutenvolumen (relativ zu einem Referenzwert); Zählwert der Atempausen (oder der Apnoe-Ereignisse); Dauer der Atempausen; Zähler der Hyperventilationsphasen; Dauer der Hyperventilationsphasen; Klassifizierung der Atemphasen (normal, obstruktive Apnoe, Central-Apnoe, Hyperventilation, Cheyne-Stokes-Atmung). Eine Überwachung von Lungenödemen ist auch denkbar.

Von dem Implantat erfasste Herzaktionssignale können auch mit den Atemsignalen korreliert werden. Das ist insbesondere wichtig für die Überwachung der zentralen Schlaf-Apnoe und für Patienten mit Herzinsuffizienz, welche an Schlaf-Apnoe leiden. Einige der Herzsignaldaten können die Herzrate, Ereigniszähler usw. enthalten, sind aber nicht auf diese beschränkt. Veränderungen der Herzrate können sich als besonders nützlich erweisen in Verbindung mit Atmungsdaten, seit bekannt ist, dass die obstruktive Schlaf-Apnoe häufig von alternierenden Phasen der Bradykardie und der Tachykardie begleitet ist. Die diagnostische Funktionalität des Implantats kann mit therapeutisches Optionen kombiniert sein, beispielsweise einer atrialen Überstimulation. Der Atmungssensor kann eine Schrittsteuerungstherapie auslösen, wenn eine Apnoe detektiert wurde.

Das Gerät kann auch als Therapiemonitor zum Einsatz kommen. Ein Langzeiterfolg oder kurzfristiger Erfolg der Schrittmachertherapie kann überwacht werden. Zusätzlich kann eine medikamentöse Therapie entsprechend einer Atemfehlfunktion in Verbindung mit dem Implantat zum Einsatz kommen.

Die Erfindung soll nun anhand von Figuren näher erläutert werden:
Figur 1 zeigt - schematisch - eine Ausführungsform des implantierbaren medizinischen Gerätes, das Zusammenwirken seiner Merkmale und seine Wirkung mit Geräten in seiner Umgebung.
Figur 2 zeigt -schematisch - Elemente der Schlaf-Detektoreinheit und der Apnoe-Detektoreinheit zusammen mit Sensoren in einem Signalverlaufsdiagramm.

Die Figuren 3, 4 und 5 zeigen Ausführungsbeispiele für Positionssensoren.

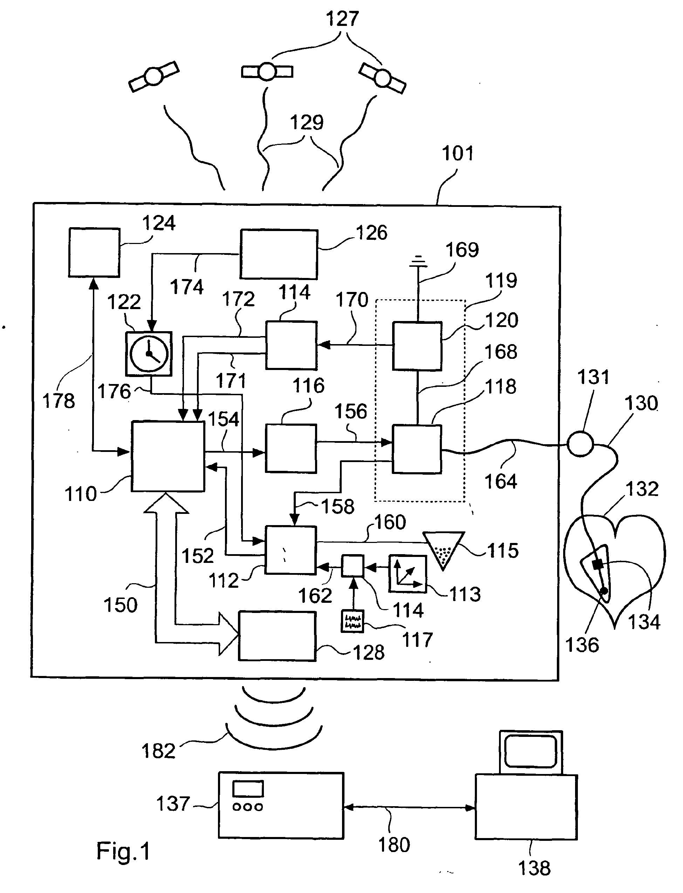

Figur 1 zeigt ein implantierbares medizinisches Gerät 101 mit einer zentralen Steuereinheit 110, einer Schlaf-Detektoreinheit 112, einer Apnoe-Detektoreinheit 114, einer Schrittmacher-Einheit 118 und einem Impedanzsensor 120. Das implantierbare medizinische Gerät 101 weist auch einen Stimulationselektrodenausgang 131 auf, welcher über eine Verbindungsleitung 164 mit der Schrittmachereinheit 118 verbunden ist. Dargestellt ist auch ein Herz 132, eine Elektrodenleitung 130, welche in den rechten Vorhof des Herzens 132 mündet und an deren distalem Endbereich eine Ringelektrode 134 und eine Spitzenelektrode 136 angebracht ist.

Der Impedanzsensor 120 ist ausgebildet, zur Erfassung einer intrathorakalen Impedanz einen Strom zwischen dem Gehäuse des implantierbaren medizinischen Gerätes 101 und der Ringelektrode 134 fließen zu lassen und eine daraus resultierende Spannung zwischen dem Gehäuse und der Spitzenelektrode 136 zu erfassen. Dazu ist der Impedanzsensor 120 über eine Gehäuseleitung 169 mit dem Gehäuse des implantierbaren medizinischen Gerätes 101 verbunden und über eine Verbindungsleitung 168 mit der Schrittmacher-Einheit 118 verbunden, wobei die Schrittmacher-Einheit 118 ausgebildet ist, während einer Stimulationspause eine elektrische Verbindung zwischen dem Impendanzsensor 120 und der Ringelektrode 134 und der Spitzenelektrode 136 herzustellen, wenn die Elektrodenleitung 130 am Stimulationsausgang 131 des implantierbaren medizinischen Gerätes 101 angeschlossen ist.

Die Apnoe-Detektoreinheit 114 ist über eine Verbindungsleitung 170 mit dem Impedanzsensor 120 verbunden und ausgebildet, den Zeitverlauf eines von dem Impedanzsensor 120 erfassten intrathorakalen Impedanzsignals auszuwerten und daraus ein Atmungssignal zu erzeugen, dieses Atmungssignal nach seinem Zeitverlauf auszuwerten und ein entsprechendes - beispielsweise Atempauseninformation enthaltendes - Auswertungsergebnis erzeugen.

Als Ausgangssignal kann die Apnoe-Detektoreinheit 114 ein Apnoe-Detektorsignal erzeugen, welches das Auswertungsergebnis beispielsweise in Form einer Apnoe-Statusinformation und einer Apnoe-Therapieinformation repräsentiert und dieses an die zentrale Steuereinheit 110 senden. Dazu ist die Apnoe-Detektoreinheit 114 ausgangsseitig über eine Verbindungsleitung 172 mit der zentralen Steuereinheit 110 verbunden. Die Apnoe-Detektoreinheit 114 ist auch zur Übersendung von Apnoe-Statistikinformationen über eine Verbindungsleitung 171 mit der zentralen Steuereinheit 110 verbunden und dazu ausgebildet, ein statistisches Auswertungsergebnis des Atemsignals zu erzeugen und ein das Auswertungsergebnis repräsentierendes Apnoe-Statistiksignal über die Verbindungsleitung 171 an die zentrale Steuereinheit 110 zu senden.

Das Apnoe-Statistiksignal kann die folgenden Parameter enthalten:

| | |
|---|---|
| Atmungsfrequenz: | Trend, Histogramm, minimum, maximum, Mittelwert, Atmungsamplitude; |
| Atem-Minutenventilation: | Trend, Histogramm, minimum, maximum, Mittelwert; |
| Apnea Ereignisse: | Absolute Anzahl, Anzahl pro Nacht; |
| Dauer der Apnea Ereignisse: | Trend, Histogramm, minimum, maximum, Mittelwert, Totale während einer Nacht; |
| Anzahl der Hyperventilationsphasen: | Absolute Anzahl, Anzahl pro Nacht; |
| Klassifizierung der Atemphasen: | normale Atmung, obstruktive Apnea, Zentrale Apnea, Hyperventilation, Cheyne-Stokes Atmung. |

Die zentrale Steuereinheit 110 ist beispielsweise als programmierbarer Mikroprozessor ausgebildet und kann ein in dieser implementiertes Steuerprogramm ausführen.

Die Schlaf-Detektoreinheit 112 ist ausgebildet, in Abhängigkeit von eingangsseitig über die Verbindungsleitungen 151, 158, 160, 162, und 176 Signale zu empfangen, diese auszuwerten, ein dem Auswertungsergebnis entsprechendes Schlaf-Signal zu erzeugen und dieses ausgangsseitig über die Verbindungsleitung 152 an die zentrale Steuereinheit 110 zu senden.

Die Schlaf-Detektoreinheit 112 ist eingangsseitig über eine Herzraten-Verbindungsleitung 158 mit der Schrittmacher-Einheit 118 verbunden, welche ausgangsseitig ein einer erfassten Herzrate entsprechendes Herzratensignal erzeugt und dieses über die Verbindungsleitung 158 an die Schlaf-Detektoreinheit 112 senden kann.

Die Schlaf-Detektoreinheit 112 ist eingangsseitig über eine Verbindungsleitung 160 mit einem Positionssensor 115 verbunden, welcher ausgebildet ist, in Abhängigkeit von seiner Winkellage im Verhältnis zur Horizontalen seinen elektrischen Widerstand zu ändern. Die Schlaf-Detektoreinheit 112 ist ausgebildet, die Winkellage des Positionssensor 115 abzufragen und dazu über die Verbindungsleitung 160 eine elektrische Spannung an den Positionssensor 115 anzulegen und einen resultierenden elektrischen Strom zu erfassen.

Die Schlaf-Detektoreinheit 112 ist eingangsseitig über eine Verbindungsleitung 162 mit einem Beschleunigungsmuster-Klassifizierer 114 verbunden, welcher mit einem triaxialen Beschleunigungssensor 113 verbunden ist. Der Beschleunigungsmuster-Klassifizierer 114 ist ausgebildet, ein Zeitsignal des triaxialen Beschleunigungssensors 113 auszuwerten und zwischen verschiedenen Beschleunigungsmustern, welche Bewegungsmustern eines Trägers des implantierbaren medizinischen Gerätes 101 entsprechen, zu unterscheiden. Dazu ist der Beschleunigungsmuster-Klassifizierer 114 über eine Verbindungsleitung mit einer Beschleunigungsmuster-Speichereinheit 117 verbunden und ausgebildet, aus dieser dort abgespeicherte Beschleunigungsmuster auszulesen und mit den über den triaxialen Beschleunigungssensor 113 erfassten Beschleunigungsmustern zu vergleichen und die erfassten Beschleunigungsmuster zu klassifizieren. Der Beschleunigungsmuster-Klassifizierer 114 kann ein ein Beschleunigungsmuster repräsentierendes Beschleunigungsmuster-Signal erzeugen und dieses über die Verbindungsleitung 162 an die Schlaf-Detektoreinheit 112 senden.

Der Beschleunigungsmuster-Klassifizierer 114 kann einen FFT-Analysator aufweisen, welcher ausgebildet ist, aus dem Zeitsignal des triaxialen Beschleunigungssensors 113 kontinuierlich eine Folge von Beschleunigungsmuster-Leistungsspektren zu erzeugen. Die Klassifizierung erfolgt dann anhand der Beschleunigungsmuster-Leistungsspektren und in der Beschleunigungsmuster-Speichereinheit 117 sind vorbestimmt Beschleunigungsmuster-Leistungsspektren abgelegt.

Alternativ zu dieser Ausführungsform kann die Beschleunigungsmuster-Klassifikation auch anhand von Zeitsignalen erfolgen, was allerdings im Vergleich zu den Beschleunigungsmuster-Leistungsspektren deutlich mehr Speicherplatz erfordert.

Die Schlaf-Detektoreinheit ist über eine Verbindungsleitung 176 mit einer Tageszeit-Uhr 122 verbunden, welche ausgebildet ist, ein einer Uhrzeit entsprechendes Tageszeit-Signal zu erzeugen und dieses ausgangsseitig über die Verbindungsleitung 176 an die Schlaf-Detektoreinheit zu senden. Die Tageszeit-Uhr ist über eine Verbindungsleitung 174 mit einem Welt-Zeitzonen-Detektor verbunden, welcher einen Satellitenempfänger, beispielsweise eine GPS-Empfänger aufweist.

Der Satellitenempfänger ist ausgebildet, ein von Satelliten 128 ausgesendetes Satellitensignal 129 zu empfangen, dieses auszuwerten und daraus eine Erdposition zu errechnen, beispielsweise in Form von Koordinaten. Der Welt-Zeitzonen-Detektor ist ausgebildet, beispielsweise mit einer Look-up-Tabelle eine ermittelte Erdposition einer Welt-Zeitzone zuzuordnen und ein Welt-Zeitzonensignal zu erzeugen und dieses ausgangsseitig über die Verbindungsleitung 174 an die Tageszeit-Uhr 122 zu senden.

Die zentrale Steuereinheit 110 ist über eine Verbindungsleitung 154 mit der Therapieeinheit 116 verbunden und kann je nach Erfordernis einer Therapie, gesteuert durch das Steuerprogramm in Abhängigkeit von dem über die Verbindungsleitung 172 empfangenen Apnoe-Detektorsignal und dem über die Verbindungsleitung 152 empfangenen Schlaf-Signal ein eine Therapieinformation enthaltendes Therapiesignal erzeugen und dieses Therapiesignal über die Verbindungsleitung 154 an die Therapieeinheit 116 senden. Die Therapieeinheit 116 ist über eine Verbindungsleitung 156 mit der Schrittmacher-Einheit 118 verbunden und ausgebildet, je nach von der zentralen Steuereinheit über die Verbindungsleitung 154 angeforderter Therapie aufgrund des Therapiesignals ein Herzraten-Anforderungssignal zu erzeugen und dieses über die Verbindungsleitung 156 an die Schrittmacher-Einheit 118 zu senden, welche daraufhin die Schrittrate entsprechend einstellen kann.

Die zentrale Steuereinheit 110 ist zur Speicherung von beispielsweise über die Verbindungsleitung 171 empfangenen Apnoe-Statistikinformationen über eine Verbindungsleitung 178 mit einer Speichereinheit 124 verbunden.

Die zentrale Steuereinheit 110 ist über einen bidirektionalen Datenbus 150 mit einer Telemetrieeinheit zur drahtlosen Datenübertragung (Long-Distance-Telemetrie-System) 128 verbunden. Beispielsweise ist die Telemetrieeinheit 128 eine Bluetooth-Telemetrieeinheit.

Die zentrale Steuereinheit 110 kann somit Apnoe-Statistikinformation sowie von der Schrittmacher-Einheit 118 erfasste Herzsignalinformation 182 über die Telemetrieeinheit 128 schnurlos an ein mobiles Patienten-Applikationsgerät 137 senden. Über die Telemetrieeinheit 128 kann ein Steuerprogramm empfangen und über den bidirektionalen Datenbus 150 an die zentrale Steuereinheit 110 gesendet und dort abgelegt werden, die zentrale Steuereinheit 110 und die Telemetrieeinheit 128 können dazu entsprechend ausgebildet sein.

Das mobile Patienten-Applikationsgerät 137 kann beispielsweise in der Nähe eines Patientenbettes aufgestellt sein. Das mobile Patienten-Applikationsgerät 137 ist zur Datenübertragung patientenbezogener Daten über eine Netzwerkverbindungsleitung 180 mit einem zentralen Servicecenter 138 verbunden. Von dort kann ein Arzt beispielsweise Patienteninformationen abrufen und überwachen. Alternativ zur Netzwerkverbindungsleitung 180 kann das mobile Patientenapplikationsgerät 137 und das zentrale Servicecenter 138 eine schnurlose Schnittstelle aufweisen, beispielsweise eine Bluetooth-Schnittstelle, und über diese Bluetooth-Schnittstelle schnurlos patientenbezogene Daten übertragen.

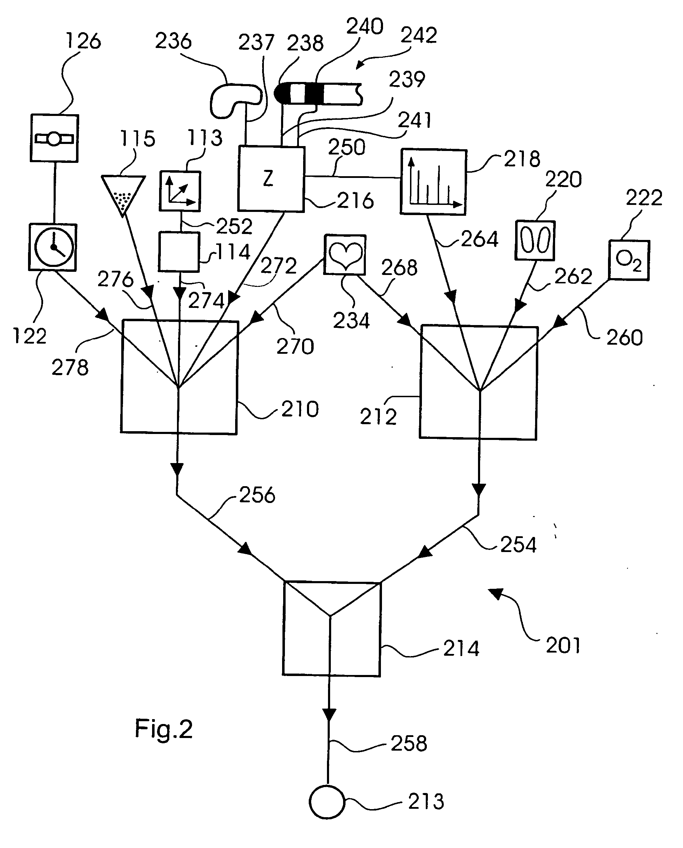

Figur 2 zeigt - schematisch dargestellt - das Zusammenwirken eines Schlaf-Gewichtungs-Diskriminators 210, welcher in der Schlaf-Detektoreinheit 112 enthalten sein kann, mit einem Apnoe-Gewichtungs-Diskriminator 212, welcher in einer Apnoe-Detektoreinheit 114 enthalten sein kann.

Der Schlaf-Gewichtungs-Diskriminator 210 weist Signaleingänge auf, an welche über Verbindungsleitungen 270, 272, 274, 276 und 278 Sensoren beziehungsweise im weiteren Sinne Signalerzeuger angeschlossen sind. Der Schlaf-Gewichtungs-Diskriminator 210 ist ausgebildet, gemäß einer vorbestimmten Gewichtungsfunktion die an den Signaleingängen anliegenden Signale zu bewerten und ein das Bewertungsergebnis repräsentierendes Schlaf-signal zu erzeugen und dieses ausgangsseitig über eine Verbindungsleitung 256 an einen Therapie-Diskriminator 214 zu senden.

Der Apnoe-Gewichtungs-Diskriminator 212 weist auch Signaleingänge auf, welche über Verbindungsleitungen 260, 262, 264 und 268 mit Signalerzeugern verbunden sind. Der Apnoe-Gewichtungs-Diskriminator 212 bewertet die an den Signaleingängen anliegenden Signale gemäß einer vorbestimmten Gewichtungsfunktion und erzeugt ein Apnoe-Signal und sendet dies ausgangsseitig über eine angeschlossene Verbindungsleitung 254 an den Therapie-Diskriminator 214.

Der Schlaf-Gewichtungs-Diskriminator 210 ist über eine Verbindungsleitung 270 mit einem Herzratensensor 234 verbunden, welcher in der Schrittmacher-Einheit 118 enthalten sein kann. Über die Verbindungsleitung 270 kann somit eine Ist-Zustands-Herzrate von dem Herzratensensor 234 ausgesendet und von dem Schlaf-Gewichtungs-Diskriminator 210 empfangen werden. Der Schlaf-Gewichtungs-Diskriminator 210 ist über eine Verbindungsleitung 272 eingangsseitig mit einem Impedanzsensor 216 verbunden. Der Impedanzsensor 216 ist über eine Verbindungsleitung 241 mit einer Ringelektrode 240 und über eine Verbindungsleitung 239 mit einer Spitzenelektrode 238 verbunden, welche im Bereich des distalen Endes 242 einer Elektrodenleitung angeordnet sind.

Der Impedanzsensor 216 ist auch über eine Verbindungsleitung 237 mit einem Gehäuse 236 des implantierbaren medizinischen Gerätes 101 verbunden und ausgebildet, ausgangsseitig über die Verbindungsleitungen 237 und 241 einen Strom fließen zu lassen und eingangsseitig über die Verbindungsleitung 239 und die Verbindungsleitung 237 eine resultierende Spannung zu erfassen und aus der erfassten Spannung und dem Strom eine Impedanz zu bilden. Der Impedanzsensor 216 ist ausgebildet, aus dieser erfassten Impedanz eine intrakardiale Impedanz zu errechnen und ein die intrakardiale Impedanz repräsentierendes Ausgangssignal über die Verbindungsleitung 272 an den Schlaf-Gewichtungs-Diskriminator 210 zu senden.

Ein bereits in Figur 1 erläuterter Beschleunigungsmuster-Klassifizierer 114 ist über eine Verbindungsleitung 252 mit einem triaxialen Beschleunigungsaufnehmer 113 verbunden und ausgebildet, ausgangsseitig ein das Klassifizierungsergebnis repräsentierende Signal über die Verbindungsleitung 274 an den Schlaf-Gewichtungs-Diskriminator 210 zu senden.

Der Schlaf-Gewichtungs-Diskriminator 210 ist auch über eine Verbindungsleitung 276 eingangsseitig mit einem - wie in Figur 1 bereits erläuterten - Positionssensor 115 verbunden.

Eine Tageszeit-Uhr 122 ist mit einem Welt-Zeitzonen-Detektor 126 eingangsseitig verbunden und ist ausgebildet, ein entsprechend einen Welt-Aufenthaltsort korrigiertes Uhrzeitsignal ausgangsseitig über eine Verbindungsleitung 278 an den Schlaf-Gewichtungs-Diskriminator 210 zu senden.

Der Apnoe-Gewichtungs-Diskriminator ist über eine Verbindungsleitung 260 eingangsseitig mit einem Blut-Sauerstoffsensor 222 verbunden, über eine Verbindungsleitung 262 eingangsseitig mit einer Atemminutenvolumen-Erfassungseinheit 220 verbunden und über eine Verbindungsleitung 264 eingangsseitig mit einer Impedanz-Auswerteeinheit 218 verbunden.

Die Impedanz-Auswerteeinheit 218 ist über eine Verbindungsleitung 250 mit dem Impedanzsensor 216 verbunden und ausgebildet, ein eine Atmungsaktivität charakterisierendes Impedanz-Zeitsignal des Impedanzsensors 216 auszuwerten und ein die Anzahl und Dauer von Atempausen charakterisierendes Atemsignal zu erzeugen und dieses ausgangsseitig über die Verbindungsleitung 264 an den Apnoe-Gewichtungs-Diskriminator 212 zu senden.
Der Apnoe-Gewichtungs-Diskriminator 212 ist über eine Verbindungsleitung 268 eingangsseitig mit dem Herzratensensor 234 verbunden und kann somit ein von diesem erzeugten Herzratensignal als Eingangsgröße empfangen.

Der Apnoe-Gewichtungs-Diskriminator ist ausgebildet, anhand einer vorbestimmten Apnoe-Gewichtungsfunktion ein Apnoe-Signal zu erzeugen und dies ausgangsseitig über die Verbindungsleitung 254 an den Therapie-Diskriminator 214 zu senden.

Der Therapie-Diskriminator bewertet anhand einer Therapie-Gewichtungsfunktion das eingangsseitig zur Verfügung stehende Apnoe-Signal und das eingangsseitig zur Verfügung stehende Schlaf-Signal und ordnet dem Bewertungsergebnis ein Therapieergebnis zu, welches von dem Therapiediskriminator ausgangsseitig über die Verbindungsleitung 258 an einem Diskriminator-Ausgang 213 ausgegeben werden kann. An den Diskriminator-Ausgang 213 kann die in Figur 1 gezeigte Therapieeinheit über die Verbindungsleitung 154 angeschlossen sein.

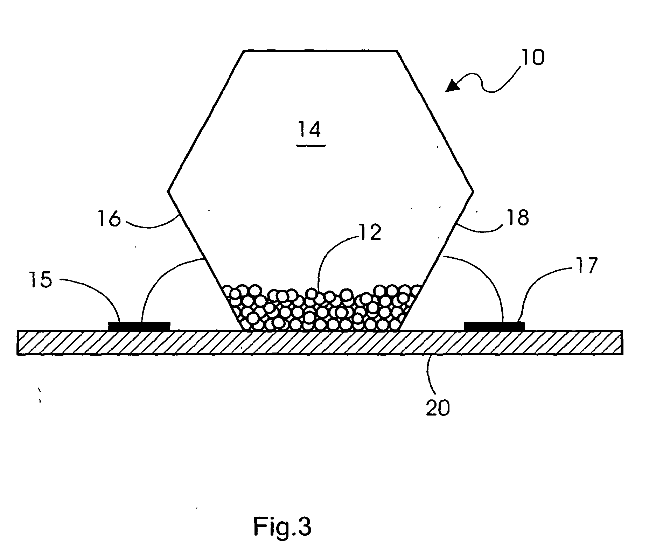

Wie in Figur 3 schematisch gezeigt, kann der Positionssensor 10 der vorliegenden Erfindung ein hexagonal geformtes Objekt mit einer Vielzahl von Metallkugeln 12 sein, welche in einem inneren Hohlraum 14 enthalten sind. Wenn der Patient liegt, ist der Positionssensor 10 derart positioniert, dass die Metallkugeln 12 eine elektrische Verbindung zwischen den unteren Seitenwänden 16 und 18 herstellen, so dass die elektrische Verbindung als Kurzschluss detektiert ist. Wenn der Patient steht, berühren die Kugeln 12 nur eine der unteren Seitenwände 16 oder 18, was als offener Schaltkreis erkannt ist. In einer gezeigten Ausführungsform sind dargestellte Kontaktflächen A und B auf einer Platine 20 angebracht. Obwohl die Verbindung der unteren Seitenwände 16 und 18 mit den Kontaktflächen als Drähte dargestellt sind, gibt es andere bekannte Möglichkeiten, um diese Verbindung herzustellen. Die Seitenwände können auch mit einer Kontaktfläche versehen sein, welche bis unter die Sockelfläche des Sensors ausgeführt ist, so dass eine einfache, verbindungsdrahtfreie Verlötung des Sensors in einem Lötwellenbad ermöglicht wird. Es ist zu erwarten, dass der Positionssensor in der Größe eines 0806-Kondensators oder eines 0603-Kondensators ausgeführt ist. Ein primärer Vorteil des Positionssensors ist, dass er keine Energie verbraucht.

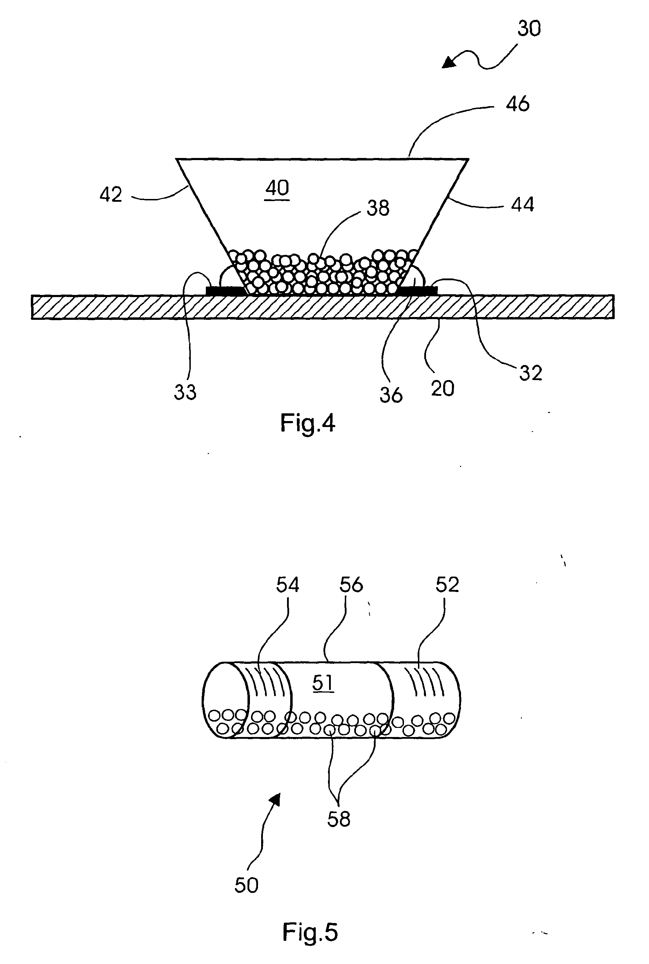

Figur 4 zeigt ein weiteres Ausführungsbeispiel für einen Positionssensor 30. Der Positionssensor 30 umfasst elektrisch leitfähige Kugeln 38, welche sich in einer Kavität 40 befinden. Die Kavität 40 wird durch elektrisch leitfähige Seitenwände 42 und 44 und durch eine Deckelfläche 46 gebildet. Anders als bei dem in Figur 3 dargestellten Positionssensor sind die Kontaktflächen 42 und 44 auch auf einem Abschnitt der zur Montage auf einer Platine vorgesehenen Bodenfläche ausgeführt. Dargestellt sind auch Kontaktflächen von Leiterbahnen 32 und 33, auf welchen jeweils eine der Kontaktflächen 42 und 44 mit Lötzinn 36 verlötet sind.

Figur 5 zeigt ein Ausführungsbeispiel eines zylindrisch geformten Positionssensors 50 mit einem Hohlraum 51, welcher von einer zylindrischen Wand 56 umschlossen ist. Die Zylinderwand 56 weist zwei längsaxial voneinander beabstandete elektrisch leitfähige Ringkontakte 54 und 52 auf. Der Hohlraum 51 ist zum Teil mit elektrisch leitfähigen Kugeln 58 angefüllt. Befindet sich der zylinderförmige Positionssensor 50 längsaxial in horizontaler Ausrichtung, so sind die elektrisch leitfähigen Ringkontakte 52 und 54 durch die elektrisch leitfähigen Kugeln 58 elektrisch miteinander verbunden. Je nach Füllstand des Hohlraums 51 mit elektrisch leitfähigen Kugeln 58 lässt sich ein Winkel zur Horizontalen einstellen, bei welchem die Ringkontakte 52 und 54 noch - oder nicht mehr - miteinander elektrisch verbunden sind.

Ist ein solcher zylinderförmiger Positionssensor 50 Bestandteil eines implantierbaren medizinischen Gerätes und ist die Längsachse des zylindrischen Positionssensors 50 parallel zur Körperlängsachse eines Trägers des implantierbaren medizinischen Gerätes ausgerichtet, so sind die elektrisch leitfähigen Ringkontakte 52 und 54 stets miteinander elektrisch verbunden, wenn der Träger des implantierbaren medizinischen Gerätes sich in einer horizontalen Position befindet, unabhängig davon, ob der Träger eine Rückenlage, Bauchlage oder Seitenlage einnimmt.

In einer nicht dargestellten Ausführungsform eines Positionssensors befindet sich eine elektrisch leitfähige Kugel in einem Hohlraum, welcher von einer Hohlkugelwand umschlossen ist. An der Innenseite der Hohlkugelwand befinden sich eine Vielzahl elektrisch leitfähiger Kontakte. Der Kugeldurchmesser der elektrisch leitfähigen Kugel in dem Hohlraum und der Abstand der elektrisch leitfähigen Kontakte auf der Innenseite der Positionssensorwand sind derart bemessen, dass in jeder beliebigen Lage des Positionssensors mindestens zwei der elektrisch leitfähigen Kontakte über die elektrisch leitfähige Kugel miteinander verbunden sind, wenn auf die elektrisch leitfähige Kugel beispielsweise eine Gewichtskraft wirkt.

## Patentansprüche

1. Medizinisches Gerät (101) zur Implantation in einen Körper, mit
einer Stimulationseinheit (118), welche ausgebildet ist, in Abhängigkeit von einem Apnoe-Therapiesignal einen elektrischen Stimulationsimpuls zu erzeugen,
einer Schlaf-Detektoreinheit (112) mit mindestens einem Signaleingang welche ausgebildet ist, in Abhängigkeit von mindestens einem Eingangssignal einen Schlafzustand des Körpers zu erkennen und ein Schlaf-Signal zu erzeugen, welches einem Schlaf-Erkennungsergebnis entspricht,
einer Apnoe-Detektoreinheit (114), welche ausgebildet ist, in Abhängigkeit von mindestens einem von dem Körper verursachten Körpersignal eine Schlaf-Apnoe zu erkennen und ein Apnoe-Signal zu erzeugen, welches einem Apnoe-Erkennungsergebnis entspricht,
einer Therapieeinheit (116), welche mit der Stimulationseinheit (118), der Schlaf-Detektoreinheit (112) und mit der Apnoe-Detektoreinheit (114) mindestens mittelbar verbunden und ausgebildet ist, in Abhängigkeit von dem Apnoe-Signal und dem Schlaf-Signal mindestens ein Apnoe-Therapiesignal zu erzeugen, welches Therapieinformation zur Prävention und/oder Behandlung von Schlaf-Apnoe repräsentiert und dieses an die Stimulationseinheit (118) zu senden,
**dadurch gekennzeichnet, dass**
das medizinische Gerät einen Positionssensor (10,30,50,115) aufweist, welcher mit dem Signaleingang der Schlaf-Detektoreinheit (112) wirkverbunden und ausgebildet ist, in Abhängigkeit von seiner Neigung um wenigstens eine durch den Positionssensor (10,30,50,115) verlaufende Raumachse im Verhältnis zur Horizontalen wenigstens eine seiner elektrischen Eigenschaften zu ändern
wobei
die Therapieeinheit (116) ausgebildet ist, das Apnoe-Therapiesignal in Form einer Zunahme der Stimulationsrate mit einer ansteigenden Flanke über eine Periode von 10 Minuten so zu erzeugen, dass sich eine Gesamtzunahme der Stimulationsrate von 10 Schlägen pro Minute durch einen Anstieg der Stimulationsrate von einem Schlag pro Minute über 10 Minuten ergibt.

2. Medizinisches Gerät nach Anspruch 1, **dadurch gekennzeichnet, dass** die Apnoe-Detektoreinheit (114) einen Apnoe-Gewichtungs-Diskriminator (212) mit mindestens einem Eingang für ein Körpersignal enthält wobei der Apnoe-Gewichtungs-Diskriminator (212) ausgebildet ist, gemäß einer vorbestimmten Apnoe-Gewichtungsfunktion das Körpersignal zu bewerten und ein eine Schlaf-Apnoe repräsentierendes Apnoe-Signal zu erzeugen.

3. Medizinisches Gerät nach einem der vorhergehenden Ansprüche, **dadurch gekennzeichnet, dass** die Schlaf-Detektoreinheit (112) einen Schlaf-Gewichtungs-Diskriminator (210) mit mindestens einem Eingang für ein Eingangssignal enthält, wobei der Schlaf-Gewichtungs-Diskriminator (210) ausgebildet ist, gemäß einer vorbestimmten Schlaf-Gewichtungsfunktion das Eingangssignal zu bewerten und ein einen Schlafzustand repräsentierendes Schlaf-Signal zu erzeugen.

4. Medizinisches Gerät nach einem der vorhergehenden Ansprüche, **dadurch gekennzeichnet, dass** das Medizinische Gerät einen Sensor zur Erfassung der intrakardialen Impedanz (216) aufweist, welcher mit dem Schlafdetektor verbunden und ausgebildet ist, anhand der intrakardialen Impedanz ein Ruhen des Körpers zu erkennen und ein den Ruhezustand des Körpers repräsentierendes Ruhezustands-Signal zu erzeugen.

5. Medizinisches Gerät nach einem der vorhergehenden Ansprüche, **dadurch gekennzeichnet, dass** das Medizinische Gerät eine Atemminutenvolumen-Erfassungseinheit (220) aufweist, welcher mit der Apnoe-Detektoreinheit (114) verbunden ist.

6. Medizinisches Gerät nach einem der vorhergehenden Ansprüche, **dadurch gekennzeichnet, dass** das Medizinische Gerät einen Sauerstoffsensor (222) aufweist, welcher mit der Apnoe-Detektoreinheit (114) verbunden und ausgebildet ist, eine Sauerstoffkonzentration im Blut zu bestimmen und ein die Blut- Sauerstoffkonzentration repräsentierendes Blut-Sauerstoffsignal zu erzeugen.

7. Medizinisches Gerät nach einem der vorhergehenden Ansprüche, **dadurch gekennzeichnet, dass** das Medizinische Gerät eine Herzschlagvolumen-Erfassungseinheit aufweist, welche mit der Schlaf-Detektoreinheit und/oder der Therapieeinheit verbunden und ausgebildet ist, ein Herzschlagvolumen zu bestimmen und ein das Herzschlagvolumen repräsentierendes Herzschlagvolumensignal zu erzeugen.

8. Medizinisches Gerät nach einem der vorhergehenden Ansprüche, **dadurch gekennzeichnet, dass** das Medizinische Gerät eine Tageszeit-Uhr (122) aufweist, welche mit der Schlaf-Detektoreinheit (112) verbunden und ausgebildet ist, ein Tageszeit-Signal zu erzeugen und dieses an die Schlaf-Detektoreinheit (112) zu senden.

9. Medizinisches Gerät nach Anspruch 8, **dadurch gekennzeichnet, dass** das Medizinische Gerät einen Welt-Zeitzonen-Detektor (126) mit einem Satellitenempfänger aufweist, wobei der Welt-Zeitzonen-Detektor (126) mit der Tageszeit-Uhr (122) wirkverbunden und ausgebildet ist, Satellitensignale (129) zu empfangen, durch Auswertung der Satellitensignale (129) eine Position auf der Erde zu ermitteln und anhand der Position auf der Erde ein Welt-Zeitzonensignal zu erzeugen und dieses an die Tageszeit-Uhr (122) zu senden, welche ausgebildet ist, die Tageszeit entsprechend zu korrigieren.

10. Medizinisches Gerät nach einem der vorhergehenden Ansprüche, **dadurch gekennzeichnet, dass** das Medizinische Gerät einen triaxialen Beschleunigungsaufnehmer (113) aufweist, bei welchem die Richtungsachsen der erfassbaren Beschleunigungen ein Orthogonalsystem bilden und welcher mit der Schlaf-Detektoreinheit (112) wirkverbunden und ausgebildet ist, ein eine Beschleunigung repräsentierendes Beschleunigungs-Zeitsignal zu erzeugen.

11. Medizinisches Gerät nach Anspruch 10, **dadurch gekennzeichnet, dass** das Medizinische Gerät einen Beschleunigungsmuster-Klassifizierer (114) aufweist, welcher ausgangsseitig mit der Schlaf-Detektoreinheit (112) und eingangsseitig mit dem Beschleunigungsaufnehmer (113) verbunden und ausgebildet ist, ein Beschleunigungs-Zeitsignal auszuwerten und vorbestimmte Beschleunigungsmuster in dem Beschleunigungs-Zeitsignal zu erkennen, diese zu klassifizieren und ein ein Beschleunigungsmuster repräsentierendes Beschleunigungsmuster-Signal zu erzeugen und dieses auszugeben.

12. Medizinisches Gerät nach Anspruch 11, **dadurch gekennzeichnet, dass** der Beschleunigungsmuster-Klassifizierer (114) eingangsseitig einen FFT-Analysator aufweist, welcher ausgebildet ist, aus einem Beschleunigungs-Zeitsignal kontinuierlich Beschleunigungsmuster-Leistungsspektren zu erzeugen und diese an den Beschleunigungsmuster-Klassifizierer zu senden.

13. Medizinisches Gerät nach einem der vorhergehenden Ansprüche, **dadurch gekennzeichnet, dass** der Positionssensor (10,30,50,115) wenigstens zwei Schaltkontakte und mindestens eine elektrisch leitfähige Kugel umfasst, welche derart angeordnet ist, in Abhängigkeit von der Neigung des Positionssensors die Schaltkontakte elektrisch leitend zu verbinden oder zu trennen.

14. Medizinisches Gerät nach einem der Ansprüche 1 bis 12, **dadurch gekennzeichnet, dass** der Positionssensor (115) eine Hallsonde ist, welche ausgebildet ist, in Abhängigkeit von ihrer Ausrichtung im Erdmagnetfeld eine Hallspannung zu erzeugen.

15. Medizinisches Gerät nach einem der vorhergehenden Ansprüche, **dadurch gekennzeichnet, dass** die Stimulationseinheit ein Herzschrittmacher oder Defibrillator ist.

16. Medizinisches Gerät nach einem der vorhergehenden Ansprüche, **dadurch gekennzeichnet, dass** die Stimulationseinheit eine Atemmuskulatur-Stimulationseinheit ist, welche ausgebildet ist, einen elektrischen Stimulationsimpuls zur Stimulation des Zwerchfells oder der Thoraxmuskulatur zu erzeugen.

17. Home-Monitoring-System mit einem implantierbaren medizinischen Gerät (101) nach einem der Ansprüche 1 bis 17 und einem mobilen Patienten-Applikationsgerät (137), wobei das mobile Patienten-Applikationsgerät (137) über eine Telemetrieeinheit (128) schnurlos mit dem implantierbaren medizinischen Gerät (101) verbunden ist.

18. Patientenüberwachungssystem mit einem implantierbaren medizinischen Gerät (101) nach einem der Ansprüche 1 bis 17 und einem mobilen Patienten-Applikationsgerät (137), welches über eine Telemetrieeinheit (128) schnurlos mit dem implantierbaren medizinischen Gerät (101) verbindbar ist und einem Zentralen-Service-Center (138), wobei das Zentrale-Service-Center (138) zur Datenübertragung patientenbezogener Daten über eine Netzwerkverbindungsleitung (180) oder eine schnurlose Verbindungsleitung mit dem mobilen Patienten-Applikationsgerät (137) wenigstens zeitweise verbunden ist.

## Claims

1. A medical device (101) for implantation in a body, having
a stimulation unit (118), which is implemented to generate an electrical stimulation pulse as a function of an apnea treatment signal,
a sleep detector unit (112) having at least one signal input, which is implemented to recognize a sleep status of the body as a function of at least one input signal and generate a sleep signal, which corresponds to a sleep recognition result,
an apnea detector unit (114), which is implemented to recognize sleep apnea as a function of at least one body signal caused by the body and generate an apnea signal which corresponds to an apnea recognition result,
a treatment unit (116), which is connected at least indirectly to the stimulation unit (118), the sleep detector unit (112), and the apnea detector unit (114), and is implemented to generate at least one apnea treatment signal as a function of the apnea signal and the sleep signal, which represents treatment information for preventing and/or treating sleep apnea, and to transmit it to the stimulation unit (118),
**characterized in that** the medical device has a position sensor (10, 30, 50, 115), which is operationally linked to the signal input of the sleep detector unit (112) and is implemented to change at least one of its electrical properties as a function of its inclination around at least one spatial axis running through the position sensor (10, 30, 50, 115) in relation to the horizontal,
the treatment unit (116) being implemented to generate the apnea treatment signal in the form of an increase of the stimulation rate having a rising flank over a period of 10 minutes in such a way that a total increase of the stimulation rate of 10 beats per minute results through a rise of the stimulation rate of one beat per minute over 10 minutes.

2. The medical device according to Claim 1, **characterized in that** the apnea detector unit (114) contains an apnea weighting discriminator (212) having at least one input for a body signal, the apnea weighting discriminator (212) being implemented to evaluate the body signal according to a predetermined apnea weighting function and to generate an apnea signal representing sleep apnea.

3. The medical device according to one of the preceding claims, **characterized in that** the sleep detector unit (112) contains a sleep weighting discriminator (210) having at least one input for an input signal, the sleep weighting discriminator (210) being implemented to evaluate the input signal according to a predetermined sleep weighting function and to generate a sleep signal representing a sleep status.

4. The medical device according to one of the preceding claims, **characterized in that** the medical device has a sensor for detecting the intracardial impedance (216), which is connected to the sleep detector and is implemented to recognize resting of the body on the basis of the intracardial impedance and to generate a rest status signal representing the rest status of the body.

5. The medical device according to one of the preceding claims, **characterized in that** the medical device has a respiratory minute volume detection unit (220), which is connected to the apnea detector unit (114).

6. The medical device according to one of the preceding claims, **characterized in that** the medical device has an oxygen sensor (222), which is connected to the apnea detector unit (114) and is implemented to determine an oxygen concentration in the blood and to generate a blood oxygen signal representing the blood oxygen concentration.

7. The medical device according to one of the preceding claims, **characterized in that** the medical device has a heartbeat volume detection unit, which is connected to the sleep detector unit and/or the treatment unit and is implemented to determine a heartbeat volume and to generate a heartbeat volume signal representing the heartbeat volume.

8. The medical device according to one of the preceding claims, **characterized in that** the medical device has a time of day clock (122), which is connected to the sleep detector unit (112) and is implemented to generate a time of day signal and transmit it to the sleep detector unit (112).

9. The medical device according to Claim 8, **characterized in that** the medical device has a world time zone detector (126) having a satellite receiver, the world time zone detector (126) being operationally linked to the time of day clock (122) and being implemented to receive satellite signals (129), to ascertain a position on the Earth by analyzing the satellite signals (129), and to generate a world time zone signal on the basis of the position on the earth and transmit it to the time of day clock (122), which is implemented to correct the time of day accordingly.

10. The medical device according to one of the preceding claims, **characterized in that** the medical device has a triaxial acceleration pickup (113), in which the directional axes of the detectable accelerations form an orthogonal system and which is operationally linked to the sleep detector unit (112) and is implemented to generate an acceleration time signal representing an acceleration.

11. The medical device according to Claim 10, **characterized in that** the medical device has an acceleration pattern classifier (114), which is connected at its output to the sleep detector unit (112) and at its input to the acceleration pickup (113) and is implemented to analyze an acceleration time signal and recognize predetermined acceleration patterns in the acceleration time signal, classify them, and generate an acceleration pattern signal representing an acceleration signal and output it.

12. The medical device according to Claim 11, **characterized in that** the acceleration pattern classifier (114) has an FFT analyzer at its input, which is implemented to generate acceleration pattern performance spectra continuously from an acceleration time signal and transmit them to the acceleration pattern classifier.

13. The medical device according to one of the preceding claims, **characterized in that** the position sensor (10, 30, 50, 115) comprises at least two switch contacts and at least one electrically conductive ball, which is situated in such a way that it electrically connects or disconnects the switch contacts as a function of the inclination of the position sensor.

14. The medical device according to one of Claims 1 through 12, **characterized in that** the position sensor (115) is a Hall probe, which is implemented to generate a Hall voltage as a function of its orientation in the Earth's magnetic field.

15. The medical device according to one of the preceding claims, **characterized in that** the stimulation unit is a cardiac pacemaker or defibrillator.

16. The medical device according to one of the preceding claims, **characterized in that** the stimulation unit is a respiration musculature stimulation unit, which is implemented to generate an electrical stimulation pulse for stimulating the diaphragm or the thorax musculature.

17. A home monitoring system having an implantable medical device (101) according to one of Claims 1 through 17 and a mobile patient application device (137), the mobile patient application device (137) being wirelessly connected via a telemetry unit (128) to the implantable medical device (101).

18. A patient monitoring system having an implantable medical device (101) according to one of Claims 1 through 17 and a mobile patient application device (137), which is wirelessly connectable to the implantable medical device (101) via a telemetry unit (128), and a central service center (138), the central service center (138) being at least temporarily connected via a network connection line (180) or a wireless connection line to the mobile patient application device (137) for data transmission of patient-related data.

## Revendications

1. Appareil (101) médical pour l'implantation dans le corps, comprenant
une unité de stimulation (118), qui est conçue pour générer une impulsion de stimulation électrique en fonction d'un signal de thérapie d'apnée,
une unité de détection de sommeil (112) avec au moins une entrée de signal qui est conçue pour détecter un état de sommeil du corps en fonction d'au moins un signal d'entrée et générer un signal de sommeil qui correspond à un résultat de détection de sommeil,
une unité de détection d'apnée (114), qui est conçue pour reconnaître une apnée de sommeil en fonction d'au moins un signal de corps provoqué par le corps et générer un signal d'apnée qui correspond à un résultat de reconnaissance d'apnée,
une unité de thérapie (116) qui est reliée au moins indirectement à l'unité de stimulation (118), à l'unité de détection de sommeil (112) et à l'unité de détection d'apnée (114), et est conçue pour générer en fonction du signal d'apnée et du signal de sommeil au moins un signal de thérapie d'apnée qui représente de l'information sur la thérapie pour la prévention et/ou le traitement de l'apnée de sommeil et envoyer ce signal à l'unité de stimulation (118),
**caractérisé en ce que**
l'appareil médical présente un capteur de position (10 30, 50, 115) qui est en liaison active avec l'entrée de signal de l'unité de détection de sommeil (112) et est conçu pour modifier au moins l'une de ses propriétés électriques en fonction de son inclinaison autour d'au moins un axe de l'espace agencé par le capteur de position (10, 30, 50, 115) par rapport à l'horizontale,
l'unité de thérapie (116) est conçue pour générer un signal de thérapie d'apnée sous la forme d'une élévation du taux de stimulation avec un flanc croissant pendant une période de 10 minutes de telle sorte qu'on obtient un accroissement global du taux de stimulation de 10 battements/minute par une augmentation du taux de stimulation de 1 battement pendant 10 minutes.

2. Appareil médical selon la revendication 1, **caractérisé en ce que** l'unité de détection d'apnée (114) contient un discriminateur de pondération d'apnée (212) avec au moins une entrée pour un signal corporel, le discriminateur de pondération d'apnée (212) étant réalisé pour évaluer le signal corporel selon une fonction de pondération d'apnée prédéfinie et générer un signal d'apnée représentant une apnée de sommeil.

3. Appareil médical selon l'une quelconque des revendications précédentes, **caractérisé en ce que** l'unité de détection de sommeil (112) contient un discriminateur de pondération de sommeil (210) avec au moins une entrée pour un signal d'entrée, le discriminateur de pondération de sommeil (210) étant conçu pour évaluer le signal d'entrée selon une fonction prédéfinie de pondération de sommeil et générer un signal de sommeil représentant un état de sommeil.

4. Appareil médical selon l'une quelconque des revendications précédentes, **caractérisé en ce que** l'appareil médical présente un capteur pour la détection de l'impédance (216) intracardiaque qui est relié au détecteur de sommeil et est conçu pour détecter un repos du corps à l'aide de l'impédance intracardiaque et générer un signal d'état de repos représentant l'état de repos du corps.

5. Appareil médical selon l'une quelconque des revendications précédentes, **caractérisé en ce que** l'appareil médical présente une unité de détection de volume respiratoire/minute (220) qui est reliée à l'unité de détection d'apnée (114).

6. Appareil médical selon l'une quelconque des revendications précédentes, **caractérisé en ce que** l'appareil médical présente un capteur d'oxygène (222) qui est relié à l'unité de détection d'apnée (114) et est conçu pour déterminer une concentration d'oxygène dans le sang et générer un signal d'oxygène dans le sang représentant la concentration d'oxygène dans le sang.

7. Appareil médical selon l'une quelconque des revendications précédentes, **caractérisé en ce que** l'appareil médical présente une unité de détection de volume de battement cardiaque, qui est reliée à l'unité de détection de sommeil et/ou à l'unité de thérapie et est conçue pour déterminer un volume de battement cardiaque et générer un signal de volume de battement cardiaque représentant le volume de battement cardiaque.

8. Appareil médical selon l'une quelconque des revendications précédentes, **caractérisé en ce que** l'appareil médical présente une horloge indiquant l'heure du jour (122) qui est reliée à l'unité de détection de sommeil (112) et est conçue pour générer un signal d'heures du jour et l'envoyer à l'unité de détection de sommeil (112).

9. Appareil médical selon la revendication 8, **caractérisé en ce que** l'appareil médical présente un détecteur de zone de fuseaux horaires universels (126) avec un récepteur satellite, le détecteur de fuseaux horaires universels (126) étant relié activement à l'horloge indiquant l'heure du jour (122) et étant conçu pour recevoir des signaux de satellites (129), pour déterminer une position sur la terre par l'analyse des signaux de satellites (129) et générer un signal de fuseau horaire universel à l'aide de la position sur la terre et envoyer ce signal à l'horloge indiquant l'heure du jour (122), qui est conçu pour autoriser l'heure du jour en conséquence.

10. Appareil médical selon l'une quelconque des revendications précédentes, **caractérisé en ce que** l'appareil médical présente un enregistreur d'accélération (113) triaxial, sur lequel les axes de direction des accélérations pouvant être enregistrés forment un système orthogonal et qui est relié activement à l'unité de détection de sommeil (112) et est conçu pour générer un signal de temps d'accélération représentant une accélération.

11. Appareil médical la revendication 10, **caractérisé en ce que** l'appareil médical présente un dispositif de classification de modèle d'accélération (114), qui est relié côté sortie à l'unité de détection de sommeil (112) et côté entrée à l'enregistreur d'accélération (113) et est conçu pour analyser un signal de temps d'accélération et détecter des modèles d'accélération prédéfinis dans le signal de temps d'accélération, pour les classer et générer un signal de modèle d'accélération représentant un modèle d'accélération et éditer ce signal.

12. Appareil médical selon la revendication 11, **caractérisé en ce que** le dispositif de classification de modèles d'accélération (114) présente côté entrée un analyseur FFT qui est conçu pour générer en continu des spectres de puissance de modèle d'accélération à partir d'un signal de temps d'accélération et envoyer ces spectres au dispositif de classification de modèles d'accélération.

13. Appareil médical selon l'une quelconque des revendications précédentes, **caractérisé en ce que** le capteur de position (10, 30, 50, 115) comprend au moins deux contacts de commutation et au moins une boule électroconductrice, qui est disposée de façon à relier de façon électroconductrice les contacts de commutation ou à les séparer en fonction de l'inclinaison de capteur de position.

14. Appareil médical selon l'une quelconque des revendications 1 à 12, **caractérisé en ce que** le capteur de position (115) est une sonde de Hall qui est conçue pour générer une tension de Hall en fonction de son orientation dans le champ magnétique terrestre.

15. Appareil médical selon l'une quelconque des revendications précédentes, **caractérisé en ce que** l'unité de stimulation est un pacemaker ou un défibrillateur.

16. Appareil médical selon l'une quelconque des revendications précédentes, **caractérisé en ce que** l'unité de stimulation est une unité de stimulation de musculature respiratoire qui est conçue pour générer une impulsion de stimulation électrique pour la stimulation du diaphragme ou de la musculature du thorax.

17. Système de "Home Monitoring" avec un appareil (101) médical implantable selon l'une quelconque des revendications 1 à 17 et un appareil d'application mobile pour patient (137), l'appareil d'application mobile pour patient (137) étant relié par une unité de télémétrie (128) sans fil à l'appareil (101) médical implantable.

18. Système de contrôle de patient comprenant un appareil (101) médical implantable selon l'une quelconque des revendications 1 à 17 et un appareil d'application mobile pour patient (137), qui peut être relié par une unité de télémétrie (128) sans fil à l'appareil (101) médical implantable et un centre principal de service (138), le centre principal de service (138) étant relié au moins temporairement pour la transmission de données spécifiques aux patients au moyen d'une ligne de liaison de réseau (180) ou d'une ligne de liaison sans fil à l'appareil d'application mobile pour patient (137).
